# EUROPEAN PATENT APPLICATION

(11) **EP 1 514 555 A1**
(43) Date of publication of application: **16.03.2005**
(21) Application number: 04254355.3
(22) Date of filing: 21.07.2004
(51) Int. Cl.: A61K 35/78, A61P 1/16, A61P 35/00

(54) **Polyherbal formulation for hepatic and splenic disorders**

(30) Priority: 21.07.2003 GB 0317021
(71) Applicant: Sahajanand Biotech Private Limited, Surat 395 003, Gujarat (IN)
(72) Inventor: Managoli, Nandkishore, Sahajanand B. Private Ltd., Surat 395003 Gujarat (IN)
(74) Representative: Gaunt, Robert John

(57) **Abstract**

A pharmaceutical or medicinal preparation which comprises a mixture of the following five herbs: Boerhavia Diffusa, Picorrhiza Kurroa, Tephrosia Purpurea, Curcuma Longa and Trigonella Foenum-Graecum, or a mixture of the active ingredients that have been extracted from those herbs. The herbal formulation of the invention is effective for the treatment of hepatic and spienic disorders, thalassemias, Wilson's Disease, hematological malignancies, and as an adjuvant to cancer therapy.

## Description

This invention relates to a new herbal formulation which has been found to be effective for the treatment of hepatic and splenic disorders.

According to this invention there is provided a pharmaceutical or medicinal preparation which comprises a mixture of the following five herbs:
Boerhavia Diffusa
Picorrhiza Kurroa
Tephrosia Purpurea
Curcuma Longa
Trigonella Foenum-Graecum
or a mixture of the active ingredients that have been extracted from those herbs. This product has been found by the inventor to be particularly effective in the treatment of hepatic and splenic disorders. The preparation is preferably formulated for administration to patients in a capsule form.

The ingredients for a typical herbal formulation according to this invention are set out in Table 1. It should be appreciated that the proportions of the individual herbs may be varied and the figures quoted in Table 1 are by way of illustration only. In particular, the proportions of one or more of the components may be varied in order to optimize the pharmacological effects produced by the formulation to suit the specific needs of patients being treated.

It is an important feature of the product of the present invention that it contains a mixture of herbs, or extracts from herbs, rather than being based on a single herb. A synergistic effect has been noticed between various ingredients. The synergistic activity is surprising and unexpected. The activities of similar herbs are combined to optimize and enhance the pharmacological effects without increasing the adverse toxic reactions (which becomes a distinct possibility if the herbs are used singly in a concentration of 100%). The advantage of a multi-drug regimen also lies in the fact that the possibility of development of drug resistance is minimized.

Preliminary clinical trials of the product of this invention have produced definite clinical evidence of improvement in the condition of patients suffering from hepatic and splenic disorders. These improvements include:
i) reduction in serum bilirubin levels together with rapid clinical recovery from jaundice without any untoward effects;
ii) protection against drug induced hepatic injury and hepatocarcinogenesis;
iii) protection against viral hepatitis;
iv) improvement in the bile flow and choleretic effect and cholagogue activity;
v) reduction in the hepatosplenomegaly in cases of leukemias, hypersplenism and other cases of hepatosplenomegaly; and
vi) improvement in the relevant biochemical parameters like SGPT, SGOT, serum bilirubin, alkaline phosphatase;
and more subjectively:
i) sense of well being;
ii) improvement in appetite;
iii) increased vigour and enthusiasm is daily activities; and
iv) improvement in the "quality of life".

The formulation of this invention is itself hepatoprotective. It may also be used as an adjuvant to conventional modes of anticancer therapy, namely radiotherapy and/or chemotherapy to reduce the hepatosplenomegaly. The formulation may be presented as a dietary supplement for patients diagnosed as having any type of hepatosplenomegaly. It may also be used to create a sense of general well being and to increase the vitality in patients diagnosed as having any type of hepatosplenomegaly; to increase the appetite, restore health and increase the lifespan of patients diagnosed as having any type of hepatosplenomegaly, to improve the ambulatory capacity in patients diagnosed as having any type of hepatosplenomegaly; and to stimulate metabolism, accelerate anabolism, promote catabolism thereby flushing the body of toxic metabolites. The hepatic clearance of substances like iron and ferritin in cases of thalassemia, and of copper in Wilson's disease is also improved, thereby reducing the metal overload in such conditions.

### Method of extraction

Each of the herbal components of the formulation were de-seeded (wherever required), ground finely to powder form and then submitted individually to conventional solvent extraction methods.
- Boerhavia Diffusa - Aqueous root extract.
- Picorrhiza Kurroa, Tephrosia Purpurea, Curcuma Longa, Trigonella Foenum-Graecum were extracted using standard extraction methods.

### Example

### A case report of a patient with hepatic disorder

- Age: :- 32
- Sex: :- Male
- Diagnosis: :- Hepatic disorder due to alcoholism
- Treatment: :- Herbal medicine according to the present invention (referred to as Hepox)
- Dosage: :- 2 capsules (450mg per capsule) three times a day

The patient was first seen on 19 February 2004, when he was examined, a Hepatic Profile Report was prepared and therapy started.

At that time, the patient was suffering from pain and tenderness over the right hypochondriac region and liver enlargement was approximately 25mm (1 inch).

| **Hepatic Profile (19/02/04)** | | |
|---|---|---|
| | **Patient Value** | **Reference Range** |
| Total Bilirubin | 2.2 | 0.2 -1.0 mg % |
| Direct | 1.0 | 0.1 -0.4 mg % |
| Indirect | 1.2 | 0.2 -0.8 mg % |

The patient's first follow-up was on 2 March 2004. On this date there was no pain, tenderness and liver enlargement was minimal.

| **Hepatic Profile (02/03/04)** | | |
|---|---|---|
| | **Patient Value** | **Reference Range** |
| Total Bilirubin | 1.3 | 0.2 -1.0 mg % |
| Direct | 0.9 | 0.1 -0.4 mg % |
| Indirect | 0.4 | 0.2 -0.8 mg % |

The patient's improvement is attributed to the administration of Hepox. The Hepox product is a preparation according to the present invention and has the following composition:-

| HEPOX | |
|---|---|
| *Ingredient* | *Proportion (by weight)* |
| Boerhavia Diffusa | 20% |
| Piccorhiza Kurroa | 20% |
| Tephrosea Purpurea | 20% |
| Curcuma Longa | 20% |
| Trigonella Foenum-Graecum | 20% |

Dosage: 450 mg/capsule

The herbal ingredients used in the above mentioned formulation are standardized hydro-alcoholic extracts strategically combined as per the percentages mentioned, and then they are encapsulated.

### Acute Oral Toxicity Test

An acute oral toxicity test (in rats) has been carried out for capsules of the Hepox formulation. A single oral administration of Hepox was given to Sprague Dawley rats to assess its acute toxicity. In the sighting studies, Hepox did not cause mortality or signs of toxicity in females treated at 300 mg or 200 mg/kg body weight. During the main study, also at 200 mg/kg, it did not cause any mortality or signs of toxicity.

Hepox did not adversely affect the body weight gain of treated rats during the 14 day observation period, post-treatment. It also did not induce any gross pathological alternations in any of the rats, as evident at necropsy.

**Table 1**

| **Polyherbal formulation for Haematological Malignancies** | | | | | | |
|---|---|---|---|---|---|---|
| ***Description of Ingredients*** | | | | | | |
| Sr. No. | Latin Binomial | Common Names | Distribution | Parts used | Quantity | Adverse Reactions |
| 1 | Boerhavia Diffusa | Punarnava | Throughout India in forests | Root | 17 - 23% preferably 20% | None |
| 2 | Tephrosia Purpurea | Sarpankho | Cultivated throughout India | Whole plant | 17 - 23% preferably 20% | None |
| 3. | Curcuma Longa | Turmeric, Haldi, Haridra | Cultivated throughout India | Rhizomes (dried as well as raw) | 17 - 23% preferably 20% | None |
| 4. | Picorrhiza Kurroa | Kadu | Cultivated throughout India | Leaves | 17 - 23% preferably 20% | None |
| 5. | Trigonella Foenum-Graecum | Methi | Cultivated throughout India | Seeds | 17-23% preferably 20% | None |

## Claims

1. A pharmaceutical or medicinal preparation comprising a mixture of the herbs: Boerhavia Diffusa, Picorrhiza Kurroa, Tephrosia Purpurea, Curcuma Longa and Trigonella Foenum - Graecum, or a mixture of the active ingredients that have been extracted from those herbs.

2. A pharmaceutical or medicinal preparation as claimed in claim 1, for use in the treatment of all types of jaundice.

3. A pharmaceutical or medicinal preparation as claimed in claim 1, for use in the treatment of hepatic and splenic disorders.

4. A pharmaceutical or medicinal preparation as claimed in claim 1, for use in the treatment of hepatosplenomegaly.

5. A pharmaceutical or medicinal preparation as claimed in claim 1, for use in the treatment of drug induced liver injury and hepatocarcinogenesis.

6. A pharmaceutical or medicinal preparation as claimed in claim 1, for use in the treatment of anaemia.

7. A pharmaceutical or medicinal preparation as claimed in claim 1, for use in the treatment of viral hepatitis.

8. A pharmaceutical or medicinal preparation as claimed in claim 1, for use in the treatment of hypersplenism.

9. A pharmaceutical or medicinal preparation as claimed in claim 1, for use as a cholagogue and choleretic.

10. A pharmaceutical or medicinal preparation as claimed in claim 1, for use as an adjuvant to conventional modes of anticancer therapy, namely radiotherapy and/or chemotherapy, and also in hematological malignancies for reducing hepatosplenomegaly.

11. A pharmaceutical or medicinal preparation as claimed in claim 1, for use in the treatment of thalasemias as an iron chelating agent.

12. A pharmaceutical or medicinal preparation as claimed in claim 1, for use in the treatment of Wilson's disease to reduce the levels of serum copper.

13. A pharmaceutical or medicinal preparation as claimed in any one of claims 1 to 12, wherein the amount of the herbs is as follows:
| Latin Binomial | Quantity |
|---|---|
| Boerhavia Diffusa | 17 - 23% preferably 20% |
| Tephrosia Purpurea | 17 - 23% preferably 20% |
| Curcuma Longa | 17 - 23% preferably 20% |
| Picorrhiza Kurroa | 17 - 23% preferably 20% |
| Trigonella Foenum- Graecum | 17 - 23% preferably 20% |

14. A pharmaceutical or medicinal preparation as claimed in any one of claims 1 to 12, wherein the amount of the herbs is as follows:
| Latin Binomial | Quantity |
|---|---|
| Boerhavia Diffusa | 20% |
| Tephrosia Purpurea | 20% |
| Curcuma Longa | 20% |
| Picorrhiza Kurroa | 20% |
| Trigonella Foenum-Graecum | 20% |

15. A dietary supplement for patients diagnosed as having any type of hepatic and splenic disorders, which includes a pharmaceutical or medicinal preparation as claimed in claim 1.
